# EUROPEAN PATENT APPLICATION

(11) **EP 4 265 587 A1**
(43) Date of publication of application: **25.10.2023**
(21) Application number: 23168614.8
(22) Date of filing: 18.04.2023
(51) Int. Cl.: C05F 11/00, C05F 17/50, C12M 1/107, C02F 3/30, C05F 17/40, C12P 5/02, C02F 11/04, C02F 103/20

(54) **METHOD FOR TREATING SUBSTRATE WITH A SIGNIFICANT NITROGEN CONTENT, ESPECIALLY CONTAINING POULTRY MANURE TO PRODUCING BIOGAS AND A METHOD FOR PRODUCING BIOGAS USING A SUBSTRATE**

(30) Priority: 19.04.2022 PL 44097322
(71) Applicant: Ignaciuk, Henryk, 21-560 Miedzyrzec Podlaski (PL); Ignaciuk, Jakub, 21-560 Miedzyrzec Podlaski (PL); Ignaciuk, Wiktor, 21-560 Miedzyrzec Podlaski (PL)
(72) Inventor: Ignaciuk, Henryk, 21-560 Miedzyrzec Podlaski (PL); Ignaciuk, Jakub, 21-560 Miedzyrzec Podlaski (PL); Ignaciuk, Wiktor, 21-560 Miedzyrzec Podlaski (PL)
(74) Representative: Kalita, Lucjan

(57) **Abstract**

The object of the invention is a processing method of nitrogen-rich substrate, particularly poultry manure, for the production of biogas involving a nitrification process, **in which** poultry manure is mixed with slurry or liquid fraction of post-fermentation sludge from biogas production, or any mixture of the above, with the weight ratios of manure dry mass to slurry dry mass and post-fermentation sludge dry mass do not exceed 84% or 87%, respectively. The substrate derived in this way is continuously mixed under oxygen supply for the process of nitrification and lowering the contents of ammoniacal nitrogen to under 3.5 g N-NH₄/dm³, which is followed by denitrification process in anaerobic conditions. When the ammoniacal nitrogen content is reduced to less than 2.6 g N-NH₄/dm³, the substrate is acidified to pH 3-4.5. The object of the invention further encompasses a method of biogas production with the use of the nitrogen-rich substrate obtained according to the above method.

## Description

The object of the invention is a processing method for nitrogen-rich substrate, particularly containing poultry manure, to be used for highly efficient and stable production of biogas. The object of the invention further encompasses a method of biogas production with the use of the substrate.

Biogas production process at biogas plants is commonly known, consisting of hydrolysis, acidogenesis, acetogenesis, methanogenesis stages in anaerobic conditions. For the above specified processes to occur properly and efficiently, an adequate carbon to nitrogen (C:N) ratio must be maintained, which is a fundamental requirement because the C:N ratio being too small may lead to methane fermentation process inhibition, particularly in the case of ammoniacal nitrogen.

As an example, from the patent application publication EP3382030A1, a biogas production method is known in a methane fermentation process consisting of hydrolysis, acidogenesis, acetogenesis and methanogenesis steps, where each step takes place in a separate chamber, with all the chambers connected together, with C:N (carbon to nitrogen) ratio for the substrate ranges from 14:1 to 40:1. In the first step, the substrate is mixed, homogenised and a batch is formed, inoculated with a bacterial inoculation derived from the digestate obtained in the process, containing hydrolysis, acidogenesis, acetogenesis and methanogenesis bacteria, subjected to aerobic treatment before inoculation. In the second stage, the batch derived in stage 1 is fed to an acid/hydrogen fermenter where the process takes place at pH values ranging from 2.5 to 4.5. In stage 3, the batch is fed in rapid cycles to a methane fermentation chamber with an elongated shape, through which the mass is transferred in a single direction from the beginning to the end of the chamber, and the process takes place within pH value range from 7.2 to 7.5 at the beginning of the chamber to 7.8 - 8.2 at the end of the chamber.

On the other hand, publication of the patent application CN103103216A presents the composition of substrate for biogas production which is obtained through mixing 70-80 kg livestock manure and poultry manure with 10-15 kg edible mushroom waste as a source of carbohydrates and carbon; hemicellulose, cellulose and lignin, biological bacteria: 10 kg, sludge from biogas production in fermentation chamber: 5 kg, where the manure and sludge is used in dried and crushed form.

Animal excrement, particularly poultry manure, is known to contain a lot of nitrogen, which determines the requirement to maintain an appropriate structure of biomass feed. Hence, the proportion of animal excrement in the substrate feed is limited because the ammonia released from animal excrement is the primary inhibitor of the anaerobic fermentation process. When manure is used as substrate for fermentation at agricultural biogas plants, its proportion does not exceed 30%; in exceptional cases where other co-substrates are used with minimal nitrogen contents, this percentage may reach 40-45%.

Among the known methods of lowering ammonia nitrogen concentration in the pulp, there is dilution of droppings with water or low nitrogen liquid; however, it causes a significant increase of costs of building a biogas plant because of the higher volume of tanks. Use of low nitrogen substrates as an addition to improve the carbon to nitrogen ratio increases the biogas plant operating costs as well. Excess nitrogen stripping technologies such as the use of sulphuric acid or thermal processing also involve added costs of using acid or increased energy demand.

Known nitrification and denitrification processes can be employed to neutralize ammonia, yet those are seldom used in biomass treatment for the purposes of biogas production. Nitrification is a two-phase ammonia and ammonium salts oxidation to nitrites and nitrates. In the first step of nitrification, ammonium ions contained in ammonia are oxidized to nitrite form through the action of nitrifying bacteria (*Nitrosomonas*); afterwards, the nitrites thus derived are transformed (oxidized) to nitrate form. Denitrification, on the other hand, consists of nitrate reduction by denitrifying bacteria, as a result of which free nitrogen is released. This process occurs mostly in anaerobic conditions, although denitrification in aerobic conditions is possible as well.

The patent application publication CN107399891A presents a method of processing livestock excrement and urine with the use of heterotrophic denitrifying bacteria to eliminate high organic matter and ammoniacal nitrogen contents. The sludge derived from this process is produced in a denitrification reaction and used thereafter as a biological pesticide for crops, whereas the supernatant and active sludge produced by a single-stage biological denitrification unit is used for anaerobic fermentation. Biogas production byproducts can be used as liquid or solid fertilizer. The method encompasses a high-performance single-phase denitrification unit, an aeration unit, a temperature control unit, a gas recovery and processing unit, and an anaerobic fermentation unit. The single-stage denitrification unit uses heterotrophic nitrifying bacteria and aerobic denitrifying bacteria. The main objective of the invention is to reduce the chemical oxygen demand (COD) and to eliminate nitrogen oxides (NOx). Production of biogas with this method, with the primary objective of the invention being the decomposition of organic matter, lacks efficiency.

Hence, the purpose of the invention is to develop a processing method for nitrogen-rich substrate, particularly containing poultry manure, involving nitrification and denitrification processes for highly efficient and stable production of biogas from that substrate. Another purpose is to propose a method of biogas production with the use of that substrate. Implementing nitrification and denitrification processes in a biogas plant is difficult yet feasible. The essential issue is to provide the right environment for the nitrification process, i.e. the right temperature, pH, sufficiently high volume of oxygen.

The essence of the processing method of nitrogen-rich substrate, particularly poultry manure, for the production of biogas involving a nitrification process, **consists in that** poultry manure is mixed with slurry or liquid fraction of post-fermentation sludge from biogas production, or any mixture of the above, with the weight ratios of manure dry mass to slurry dry mass and post-fermentation sludge dry mass do not exceed 84% or 87%, respectively. The substrate derived in this way is continuously mixed under oxygen supply for the process of nitrification and lowering the contents of ammoniacal nitrogen to under 3.5 g N-NH₄/dm³. This is followed by a denitrification process in anaerobic conditions; when the ammoniacal nitrogen content is reduced to less than 2.6 g N-NH₄/dm³, the substrate is acidified to pH 3-4.5.

Preferably, a selection of pig or cattle slurry or any mixture of the two is used.

Preferably, methane bacteria are eliminated from the liquid fraction of post-fermentation sludge from biogas production before mixing with manure.

Preferably, the process temperature ranges from 32 to 38_{°}C.

Preferably, nitrification takes place with the use of nitrifying bacteria: *Nitrosomonas* followed by *Nitrobacter.*

Preferably, denitrification takes place with the use of denitrifying bacteria: *Pseudomonas stutzeri* and/or *Bacillus spirillum.*

Preferably, the denitrification process takes place over 2 to 26 hours.

The essence of the method of biogas production using nitrogen-rich substrate **consists in that** the nitrogen-rich substrate is derived with the above specified method and, in addition to that, a carbon substrate is prepared separately, with C:N ratio above 30:1, which is inoculated with hydrolytic bacteria. The nitrogen-rich substrate thus derived, together with the carbon substrate, is then placed in a closed acid/hydrogen chamber and the batch is subjected to hydrolysis, acidogenesis and acetogenesis processes at 32-38_{°}C, pH 3-4.5. After 6-24 hours, the produced pulp is pumped in cycles at approx. 1.5-2.5 hour intervals to a methane fermentation tank where methanogenesis occurs at 41-45°C, pH 7-8.2.

Preferably, the proportion of nitrogen-rich substrate in the dry mass of the batch is at 65-80%.

The object of the invention is presented in detail on the sample execution models and drawings where:
Fig1 presents the pH value curves for the particular mixtures; key: OB+W - chicken manure and water; OB+W+Aeration - chicken manure and water, aerated; OB +CP - chicken manure and liquid post-fermentation sludge; OB +CP +Aeration: chicken manure and liquid post-fermentation sludge, aerated; OB+G: chicken manure and slurry, stored in anaerobic conditions; OB+G+Aeration: chicken manure and slurry, aerated,
Fig.2 presents nitrate and nitrite (N-NOx) value curves for the particular mixtures; key: see above,
Fig.3 presents the progress of the fermentation process for a mixture of chicken manure with slurry in the aeration option.

### Example 1 - Substrate treatment method

Poultry manure (chicken manure) was used as nitrogen-rich substrate for the production of biogas; it was obtained from a poultry house with straw pellets used as mulch. Physical and chemical tests of the chicken manure were carried out. The analytic tests were conducted in accordance with the Polish standards: pH (PN-90 C-04540/01), dry matter (PN-75 C-04616/01), dry organic matter and ash (PN-Z-15011-3), mineral nitrogen using Bremner's distillation method (N-NH₄ with magnesium oxide, N-NOx with Devarda's alloy), whereas C/N ratio was determined using Flash 2000 macroanalyzer in samples dried at 60°C. The manure removed after the end of breeding contained 50% dry weight. The tests demonstrated that it contained 4.754% total nitrogen in dry mass and 43.734% C in dry mass. As a consequence, the C:N ratio for the tested poultry manure was in a very narrow range of 9.2. Such high content of nitrogen compared to carbon is typical of poultry excrement and as such constitutes a fundamental issue in using poultry manure as substrate for biogas plants, due to the risk of ammonia inhibition, weakening or even withholding the process with ammoniacal nitrogen concentration in the fermenting pulp exceeding 2.6 g N-NH₄⁺ /dm³.

The poultry manure was prepared for testing by mixing it 1:3 by weight with pig manure and in the same proportion with the liquid fraction of post-fermentation sludge, which was previously aerated for the purpose of methane bacteria inhibition. The proportion of manure dry mass to slurry dry mass was at approximately 80% by weight. For comparison and to demonstrate efficiency, further tests were carried out in which the manure was mixed in the same proportions with water. Post-fermentation sludge can be derived from methane fermentation, such as the process described in publication EP3382030A1. The function of the added liquids was to liquefy the poultry manure to a form in which it could be aerated and pumped, i.e. to 10-15% dry mass.

The mixtures were then placed in reactors as follows:
- manure + water, no aeration (anaerobic conditions),
- manure + water, with aeration (aerobic conditions),
- manure + pig slurry, no aeration (anaerobic conditions),
- manure + pig slurry, with aeration (aerobic conditions),
- manure + liquid fraction of post-fermentation sludge, no aeration (anaerobic conditions),
- manure + liquid fraction of post-fermentation sludge, with aeration (aerobic conditions).

The parameters of the substrates used for testing are presented in Table 1.

**Table 1**

| Mixture | pH | N-NH₄ g/kg fresh mass | N-NOₓ g/kg fresh mass |
|---|---|---|---|
| Manure + water | 5.43 | 3.29 | 0.01 |
| Manure + liquid post-fermentation sludge | 6.87 | 4.49 | 0.05 |
| Manure + slurry | 7.11 | 5.19 | 0.04 |

After the mixtures of manure with pig slurry, prepared liquid fraction of post-fermentation sludge and water were put in reactors, they were stored in anaerobic conditions or continuously stirred and aerated to support the nitrification processes for approximately 8 hours. The process took place at 35_{°}C. It was determined that the bacteria involved in the nitrification process were the *Nitrosomonas* bacteria, converting ammonium salts and ammonia to nitrites, followed by *Nitrobacter* converting nitrates to nitrites.

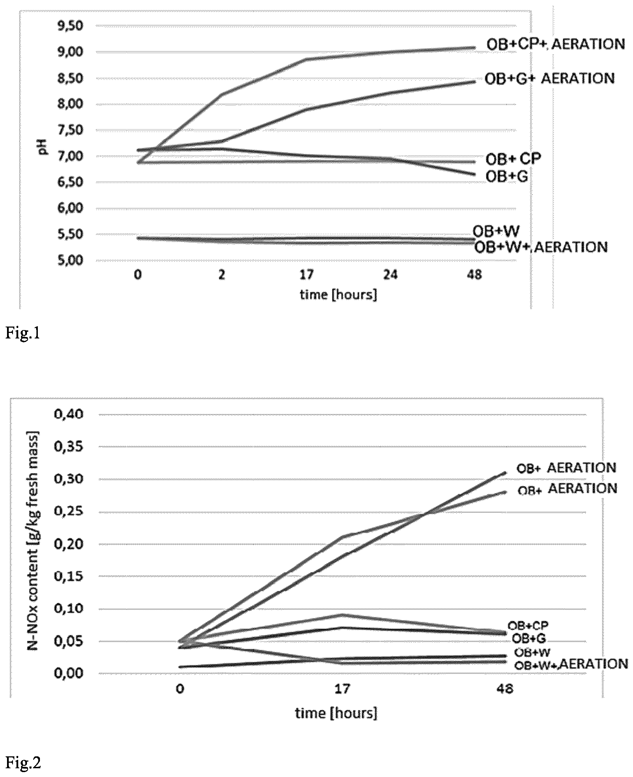

The initial pH values of the manure mixtures varied significantly. The reaction value was lowest for the manure + water mix and highest for the mix with pig slurry. When the mixtures were placed in reactors and the aeration system was activated in three of them, the differences in pH values among the mixtures began to increase even further. These variations are presented on the diagram - Fig. 1. The tests indicate that from the perspective of fitness for the fermentation process, aerated chicken manure mixes (excluding the mixture with water) are characterized by high pH values.

The nitrate and nitrite content trends were similar to those occurring in pH values, as shown on the diagram - Fig. 2. The tests recorded fluctuation of ammoniacal nitrogen being the primary inhibitor of the methane fermentation process on chicken manure. These fluctuations over the span of the experiment, together with the reference of the end N-NH₄ content to the concentration limit causing inhibition by ammonia (2.6 g/litre), are presented in Table 2 below.

**Table 2**

| Mixture | beginning [g/litre] | End [g/litr] | N-NH4 limit [g/litre] | Possible manure share [%] |
|---|---|---|---|---|
| OB+W Storage | 3.29 | 3.27 | 2.60 | 79.63 |
| OB+W Aeration | 3.29 | 2.50 | 2.60 | 104.14 |
| OB+CP Storage | 4.49 | 4.43 | 2.60 | 58.76 |
| OB+CP Aeration | 4.49 | 2.96 | 2.60 | 87.83 |
| OB+G Storage | 5.19 | 5.30 | 2.60 | 49.06 |
| OB+G Aeration | 5.19 | 3.06 | 2.60 | 84.91 |

With an aerobic environment provided, the contents of ammoniacal nitrogen in the tested mixtures could be significantly reduced. The lowest ratio (2.5 g/litre) occurred in the aerated mixture with water. However, the addition of water is impractical at a large scale, due to efficiency requirements and high prices.

Aerated manure + slurry + liquid fraction of post-fermentation sludge, on the other hand, demonstrated a major decrease of N-NH₄ contents, to 3.06 and 2.96 g/litre, respectively. With such relatively low ammoniacal nitrogen content in a blend intended for fermentation, the possible proportion of chicken manure in the substrate mix is at 87.83% weight for manure mixed with post-fermentation pulp and 84.91% for manure mixed with pig slurry, respectively.

Hence, the values derived here are significantly higher than the typical proportion of chicken manure in the flow of substrates designed for fermentation at agricultural biogas plants.

To reduce the N-NH₄ content to under 2.60 g/l, the mixtures were placed in a closed tank in order to provide anaerobic conditions and proceed with the denitrification process. Denitrification was carried out for 12 hours, with the use of *Pseudomonas stutzeri* and *Bacillus spirillum* denitrifying bacteria. After that time, ammoniacal nitrogen value decreased to 2.57 g N-NH₄/dm³. At that point, the substrate was acidified to pH =4. Duration of the denitrification process depends on the tested ammoniacal nitrogen content and should be interrupted when the latter value falls below 2.60 g N-NH₄/dm³. Continued denitrification would cause the *Pseudomonas stutzeri* and *Bacillus spirillum* bacteria engaged in the process to use the organic carbon resources in the substrate for further conversion of nitrites into gaseous nitrogen. Organic carbon, however, is necessary for production of methane. Therefore, the denitrification process needs to be effectively stopped, which is done by lowering the pH level. The value derived at 2.57 g N-NH₄/dm³ is close to the threshold (2.6 g/litre), yet in practice this limit is relatively fluid and methane bacteria are actually capable of functioning at a level even slightly exceeding 2.6 g N-NH₄/dm³. Due to the deep reduction of nitrogen content in chicken manure, the latter can be used in proportions reaching approx. 80-82% of the entire substrate flow.

After the end of the process, the methane fermentation process was tested on the prepared mixtures.

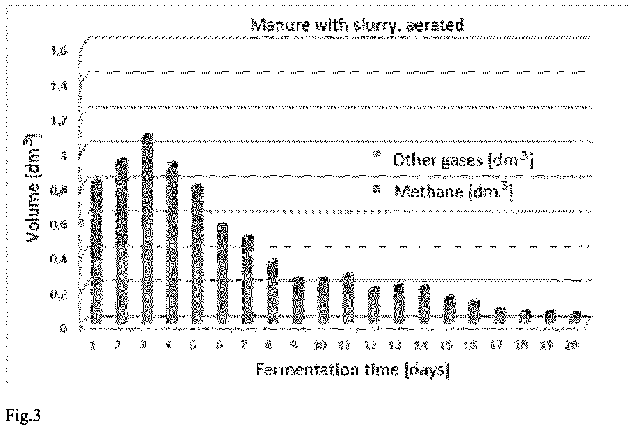

The results of the fermentation process tests demonstrated that stable biogas production was only possible with the use of the chicken manure with slurry mixture or the chicken manure with the liquid fraction of post-fermentation sludge in the storage + aeration option. Fig.3 shows a methane production process diagram for the mixture of chicken manure with slurry. The methane production process had the same characteristics for the mixture of chicken manure with liquid fraction of post-fermentation sludge.

### Example 2 - Biogas production method

The substrate was derived using a nitrification chamber with an agitator to which air with oxygen was supplied, as well as a denitrification chamber to which the pulp is pumped after the end of the nitrification process. The substrate was prepared by mixing chicken manure with pig slurry in the proportions given in example 1 and applying the processes specified there.

A carbon-rich substrate with C:N ratio exceeding 30:1 was placed in the mixing and inoculation chamber at the same time. A distillery decoction or fruit pomace can be used, for example, as such substrate. Apple pomace and corn silage was used in the case under consideration. The carbon substrate was mixed and after obtaining approx. 12% dry mass, the pulp was inoculated with hydrolytic bacteria. The duration of the process was approximately 4 hours. From the mixing and inoculation chamber, the pulp was pumped directly to the acid/hydrogen chamber, together with the chicken manure substrate from the denitrification chamber.

Considering the carbon substrates scheduled for use, the sample composition and dry mass proportion of the particular substrates in the total batch expressed as dry mass, in the option with maximised use of chicken manure, is determined as follows:
- poultry manure - 71% of the batch
- slurry - 18% of the batch
- apple pomace - 6% of the batch
- corn silage - 5% of the batch.

In the acid/hydrogen reactor, biomass was subjected to hydrolysis, acidogenesis and acetogenesis processes at 35_{°}C, pH value at approx. 4. A significant amount of acetic acid was produced after 10-12 hours. Production of significant amounts of acetic acid makes it possible to change 100% of substrate composition every day. The produced pulp was pumped in cycles at approx. 2-hour intervals, to a methane fermentation tank where methanogenesis was occurring. Methanogenesis took place at 43_{°}C, pH 7-8.2. Pumping pre-fermented pulp from the acid/hydrogen chamber to the methane fermentation chamber triggers a very strong biomass decomposition process, which occurs in just a few minutes after the batch application. The biological reactions lead to intensive production of biogas, containing mostly CH₄ and CO₂. In this reaction, the pressure in the tank increases according to the quantity of dosed pulp, from 120 to 180 mbar.

Efficient and stable biogas production can be carried out in the same way with the use of the liquid fraction of post-fermentation sludge as liquefying agent.

## Claims

1. A processing method of nitrogen-rich substrate, particularly poultry manure, for the production of biogas involving a nitrification process, **characterised in that** poultry manure is mixed with slurry or liquid fraction of post-fermentation sludge from biogas production, or any mixture of the above, with the weight ratios of manure dry mass to slurry dry mass and post-fermentation sludge dry mass do not exceed 84% or 87%, respectively; the substrate derived in this way is continuously mixed under oxygen supply for the process of nitrification and lowering the contents of ammoniacal nitrogen to under 3.5 g N-NH₄/dm³, which is followed by denitrification process in anaerobic conditions; when the ammoniacal nitrogen content is reduced to less than 2.6 g N-NH₄/dm³, the substrate is acidified to pH 3-4.5.

2. Processing method according to claim 1, **characterised in that** a selection of pig or cattle slurry or any mixture of the two is used.

3. Processing method according to claim 1, **characterised in that** methane bacteria are eliminated from the liquid fraction of post-fermentation sludge from biogas production before mixing with manure.

4. Processing method according to claim 1, or 2, or 3, **characterised in that** the process temperature ranges from 32 to 38_{°}C.

5. Processing method according to claim 1, or 2, or 3, or 4, **characterised in that** nitrification takes place with the use of nitrifying bacteria: *Nitrosomonas* followed by *Nitrobacter.*

6. Processing method according to claim 1, or 2, or 3, or 4, or 5, **characterised in that** denitrification takes place with the use of denitrifying bacteria: *Pseudomonas stutzeri* and/or *Bacillus spirillum.*

7. Processing method according to claim 1, or 2, or 3, or 4, or 5, or 6, **characterised in that** denitrification takes place for 2 to 26 hours.

8. Biogas production method using nitrogen-rich substrate, **characterised in that** the nitrogen-rich substrate is derived with the method specified in claims 1-7, and in addition to that, a carbon substrate is prepared separately, with C:N ratio above 30:1, which is inoculated with hydrolytic bacteria; the nitrogen-rich substrate thus derived, together with the carbon substrate, is then placed in a closed acid/hydrogen chamber and the batch is subjected to hydrolysis, acidogenesis and acetogenesis processes at 32-38_{°}C, pH 3-4.5; after 6-24 hours, the produced pulp is pumped in cycles at approx. 1.5-2.5 hour intervals to a methane fermentation tank where methanogenesis occurs at 41-45_{°}C, pH 7-8.2.

9. Biogas production method according to claim 8, **characterised in that** the proportion of nitrogen-rich substrate in the dry mass of the batch is at 65-80%.
